# EUROPÄISCHE PATENTSCHRIFT

(11) **EP 1 690 093 B1**
(45) Veröffentlichungstag und Bekanntmachung des Hinweises auf die Patenterteilung: **11.08.2010**
(21) Anmeldenummer: 04819210.8
(22) Anmeldetag: 23.11.2004
(51) Int. Cl.: G01N 33/543

(54) **SPHÄRISCHE, MAGNETISCHE SILICAGEL-TRÄGER MIT VERGRÖSSERTER OBERFLÄCHE FÜR DIE AUFREINIGUNG VON NUKLEINSÄUREN**
SPHERICAL AND MAGNETIC SILICAGEL CARRIERS HAVING AN INCREASE SURFACE FOR PURIFYING NUCLEIC ACIDS
SUPPORTS DE SILICAGEL MAGNETIQUES SPHERIQUES, A SUPERFICIE ACCRUE POUR PURIFIER LES ACIDES NUCLEIQUES

(30) Priorität: 25.11.2003 DE 10355409
(43) Veröffentlichungstag der Anmeldung: 16.08.2006
(73) Patentinhaber: MagnaMedics GmbH, 52066 Aachen (DE)
(72) Erfinder: MÜLLER-SCHULTE, Detlef, 52066 Aachen (DE)
(74) Vertreter: Mann, Volker
(86) Internationale Anmeldenummer: PCT/EP2004/013260
(87) Internationale Veröffentlichungsnummer: WO 2005/052581

(56) Entgegenhaltungen:
- DE-A1- 10 035 953
- US-A1- 2003 148 101
- US-B1- 6 545 143
- PATENT ABSTRACTS OF JAPAN Bd. 1998, Nr. 13, 30. November 1998 (1998-11-30) -& JP 10 214710 A (TOSOH CORP), 11. August 1998 (1998-08-11)

## Beschreibung

Die vorliegende Erfindung betrifft magnetische, (Halb-) Metalloxide-enthaltende, sphärische Silicagel-Partikel mit hoher Nukleinsäure-Bindungskapazität, ein Verfahren zu deren Herstellung sowie deren Verwendung im Bereich der Bioanalytik und Diagnostik.

Seit Jahren werden Silica-Partikel in der Bioanalytik zur Abtrennung und Aufreinigung von Nukleinsäuren verwendet, die aufgrund ihrer speziellen physikalisch-chemischen Struktur besonders dazu befähigt sind, diese Nukleinsäuren zu binden. Solche ausschließlich in der Säulenchromatographie einsetzbaren Medien sind in der Deutschen Patentschrift DE 32 11 309 (entsprechend US 4,699,717) beschrieben.

In der PCT Anmeldung EP99/08996 werden glasbeschichtete Pigmente zur Nukleinsäureaufreinigung beschrieben, die verschiedene Metalloxide wie Zink-, Bor-, Eisen-, Kalzium-, Kalium- und/oder Aluminium enthalten. Glaspartikel mit einem Glimmerkern und inkorporierten Magnetitpartikeln, die jedoch zu einer schnellen Sedimentation neigen, gehen aus der PCT Anmeldung EP96/02459 hervor. Die Herstellungsverfahren sind zeitaufwendig und erfordern technisch aufwendige Sprüh-Trocknungsverfahren. Ideal sphärische Partikel lassen sich mit diesen Verfahren nicht herstellen.

In Anal. Biochem. 201, 166 (1992) bzw. PCT GB91/00212 sind Nukleinsäure-Separationsverfahren mit Hilfe von Magnetpartikeln beschrieben, die in der Lage sind die Nukleinsäuren nach einer Salz-Ethanol-Ausfällung zu absorbieren. Diese Verfahren arbeiten jedoch nicht nukleinsäurespezifisch, d.h., die Magnetpartikel absorbieren auch parallel andere Biosubstanzen.

Silanisierte Eisenoxid-Partikel zur Immobilisierung von Enzymen sind aus der US Patentschrift 4,152,210 bekannt. Ebenfalls zum Zwecke der Enzymimmobilisierung sind in der US-Patentschrift 4,343,901 ferromagnetische Partikel beschrieben, die durch eine Sol-Gel-Technik hergestellt werden.

In der PCT-Anmeldung EP97/04828 werden monodisperse Magnetpartikel beschrieben, die aus einem SiO₂ Kern bestehen, der durch Beschichten mit Eisenoxid magnetische Eigenschaften erhält. Durch anschließende Silansierung der Eisenoxid-Schicht sind die Teilchen befähigt, Nukleinsäuren zu binden. Analog dazu sind aus der US-Patentschrift 5,320,944 0,2-3 µm große Magnetpartikel bekannt, die durch Beschichten eines Polymerpartikels mit Eisenoxiden magnetische Eigenschaften erhalten. Durch weitere Beschichtung der Partikel mit Silanen, Nylon oder Polystyrol können anschließend Antikörper für den Einsatz in Immunoassays an die Partikel gekoppelt werden. Mit kolloidalem SiO₂ beschichtete Eisenoxid Partikel sind in der US-Patentschrift 4,280,918 offenbart.

Magnetische Silica-Hybrid-Partikel, bestehend aus einem Polystyrol Kern, auf den Magnetit und anschließend eine Silica-Schicht aufpolymerisiert wird, sind aus PCT/US 95/12988 bekannt. Die Partikel werden für die Antikörper- und Zellseparation eingesetzt.

20-100 µm große magnetische Silicagel-Partikel zur Enzymimmobilisierung, die durch elektrostatische Beschichtung von Nickel-Pulvern mit Silica-Solen erzeugt werden, sind von Goetz et al., Biotechn. & Bioengineering, Vol. 37, 614, 1991, beschrieben worden.

Organosilanisierte kolloidale Silicagel-Partikel als biologische Separationsmedien sind in der PCT-Anmeldung US99/00403 offenbart, wobei die Stabilität der Kolloide und Art und Weise der Silanisierung im Vordergrund stehen. Magnetpartikel, die ein magnetisches Kernmaterial enthalten und mit einem anorganischen Oxid beschichtet sind, werden in EP 0343 934 offenbart.

Polymerpartikel, die mit einer magnetische Substanzen enthaltenden Polymerschicht beschichtet sind, auf der ein dritter zur Interaktion mit Biomolekülen befähigter Polymerüberzug aufgetragen ist, sind in der PCT Anmeldung FR97/00912 beschrieben.

10-60 µm große Perlglanzfarbpigmente, die mit Magnetit ummantelt und zur Trennung von biologischen Gemischen vorgesehen sind, gehen aus der PCT-Anmeldung DE97/01300 (korrespondierend US 6,372,517) hervor.

Magnetische Hybridpartikel, die aus einem Polymerkern bestehen, die zunächst mit einem Ferrofluid beschichtet und anschließend mit einem funktionellen Polyacrylat beschichtet werden, sind Gegenstand der US-Patentschrift 5,648,124. In den US-Patenten 6,204,033 und 6,514,688 werden sphärische, magnetische Polymerpartikel auf der Basis von Polyvinylalkohol beschrieben, die mittels inverser Suspensionspolymerisation innerhalb kurzer Zeit herstellbar sind. Die dort offenbarten Polymerpartikel sind jedoch aufgrund der chemisch-physikalischen Eigenschaften des Polyvinylalkohols ohne umfangreiche Derivatisierungsschritte nicht zur Nukleinsäure-Aufreinigung geeignet.

Die aus dem Stand der Technik bekannten Partikel weisen in bezug auf die Abtrennung von Nukleinsäuren, sofern sie überhaupt für diese Anwendung geeignet sind, einige Nachteile auf: Zum einen sind eine Reihe von Trägermedien nicht magnetisch (US 4,927,750; DE 32 11 309; PCT/US99/00403; PCT/EP94/01378), so dass eine rasche Abtrennung der Partikel, wie heute bei automatisierten Routineanalysen gefordert, nicht möglich ist. Zum anderen weisen Magnetpartikel auf Silica- oder Polystyrol-Basis, die mit einem magnetischen Oxid beschichtet sind, eine hohe spezifische Dichte auf (PCT/EP97/04828, US 4,152,210, EP 3211309, 5,320,944), woraus eine unzureichende Dispergierbarkeit verbunden mit einem schnellen Absetzen der Teilchen resultiert. Der Einsatz dieser Teilchen in einem Immuno-oder Nukleinsäureassay, der vorwiegend in Suspension durchgeführt wird, ist dadurch nachhaltig beeinträchtigt, da man auf eine zusätzliche mechanische Durchmischung angewiesen ist. Der entscheidende Nachteil der beschichteten Partikel besteht jedoch darin, daß die Metalloxide sowohl als Kernmaterial als auch als Beschichtungsmaterial trotz der anschließenden Silanisierung direkt mit der Analysenlösung in Kontakt kommen können. Dies stellt ein gravierendes Problem bei der Nukleinsäureanalytik, z.B. im Rahmen der PCR, dar, da die bei der PCR benutzten Polymerasen im Kontakt mit Metallen deaktiviert werden können.

Die aus dem Stand der Technik bekannten Verfahren zur Herstellung der Magnetpartikel sind grundsätzlich sehr aufwendig und bedürfen ohne Ausnahme eines mehrstündigen Herstellungsprozesses.

Weiterhin wird in der PCT-Anmeldung EP01/08392, entsprechend DE 100 35 953 A1, ein inverses Suspensionsverfahren zur Herstellung von Silica-Partikeln offenbart, das die aus dem Stand der Technik offensichtlichen Nachteile in bezug auf die stofflichen Gegebenheiten und/oder den zeitlichen und experimentellen Aufwand zu umgehen vermag. Die Basis dieses Verfahrens, auf das hier vollständig inhaltlich Bezug genommen wird, sind Magnetkolloid-enthaltende wäßrige Silica-Sole, die in speziellen organischen Phasen dispergiert werden und während des Dispergiervorganges durch Basenzugabe zu sphärischen Gel-Partikeln verfestigt werden. Der Nachteil der nach diesem Verfahren hergestellten Silicagele ist jedoch, dass es sich bei Ihnen um Hydrogele handelt, die infolge des hohen Wassergehaltes sehr polar bzw. hydrophil sind, was die Nukleinsäurebindungs-Kapazität nachhaltig beeinträchtigt. Zudem sind keine, spezifisch die Nukleinsäurebindung unterstützende Trägermodifikationen beschrieben, so daß ein befriedigender Einsatz der Träger bei der Nukleinsäureaufreinigung aufgrund der geringen Bindungskapazität nicht gegeben ist.

Ausgehend von diesem Stand der Technik besteht die Aufgabe der vorliegenden Erfindung darin, für die Nukleinsäureaufreinigung geeignete Silicagel-Partikel und Verfahren zu ihrer Herstellung bereitzustellen, die die Nachteile der bekannten Silica- und Polymerträger im Hinblick auf die präparationsintensiven und zeitaufwendigen Beschichtungstechniken überkommen und eine effiziente Herstellung magnetischer Partikel auf Silicagel-Basis ermöglichen.

Die nach dem erfindungsgemäßen Verfahren hergestellten sphärischen Silicagel-Partikel, die einen Gehalt an Magnetpartikeln aufweisen, und in die SiO₂-Kolloid und Metalloxide eingekapselt sind, besitzen eine deutlich vergrößerte Oberfläche und verfügen über Polymereigenschaften, die es gestatten, Nukleinsäuren in signifikanter, hoher Konzentration (> 20 mg/g Träger) zu binden.

Die Präparation der erfindungsgemäßen Partikel geht von vorgeformten wäßrigen Silica-Hydrosolen aus, die mit magnetischen Kolloiden oder Magnetpartikeln vermischt werden und anschließend in heterogener Phase unter Basenzugabe zu sphärischen Polymerpartikeln polykondensiert werden. Zur weiteren Verbesserung der Eigenschaften kann sich eine Wärmebehandlung der Polymerpartikel anschließen.

Die Herstellung der bei der Herstellung eingesetzten Silica-Sole (Hydrosole) erfolgt nach den bekannten Verfahren durch Hydrolyse von Alkoxysilanen mit Hilfe verdünnter Mineralsäuren oder organischen Säuren wie z.B. Essigsäure oder Ameisensäure. Die Alkoxysilane werden in Wasser dispergiert und durch Säurezugabe hydrolysiert, wobei zur Beschleunigung des Hydrolysevorgangs vorzugsweise Ultraschall eingesetzt wird, was auch zur besseren Durchmischung der zunächst heterogenen Phase beiträgt.

Als Alkoxysilane kommen Kieselsäureorthoester aliphatischer Alkohole wie z.B. Methyl-, Ethyl- oder Propylester einzeln oder als Mischungen zum Einsatz. In der Folge findet eine Kondensation zu niederpolymeren Silica-Hydrosolen statt, die nach und nach durch weitere Polykondensation zu mehr oder weniger viskosen Solen führen. Je nach Zusammensetzung reichen Beschallungszeiten von 5 bis 30 Minuten aus, wobei die Beschallungszeiten allgemein mit zunehmender Säurekonzentration abnehmen. Die vorzugsweise zur Hydrolyse eingesetzten Mineralsäuren weisen eine Konzentration von 0,02 bis 1 Mol/Liter auf, wobei der Volumenanteil der Säuren im Ansatz 10-35 %, vorzugsweise 20-28 %, beträgt. Die Carbonsäuren werden als reine Säuren eingesetzt; ihr Volumenanteil beträgt in der Regel 15-40 %.

Die Zusammensetzung des Gels wird entscheidend von der Art und Weise der Hydrolyse und Polykondensation bestimmt. So führt die Säurekatalyse im allgemeinen zu höheren Hydrolyseraten unter verlangsamter Polykondensation, während umgekehrt die Zugabe von Basen die Polykondensation fördert.

Die Steuerung der Hydrolyse und der Polykondensation, die in bekannter Weise dazu genutzt werden kann (vgl. PCT/EP01/08392), die Porenstruktur der Gele gezielt zu verändern bzw. einzustellen, reicht jedoch nicht aus, um eine solche Oberflächenvergößerung herbeizuführen, die es ermöglicht, eine signifikante Menge Nukleinsäure zu binden. Diese Oberflächenvergrößerung wird überraschenderweise dadurch erreicht, dass dem Silica-Sol vor der Suspension ein vorgefertigtes SiO₂-Kolloid zugesetzt wird, dessen Teilchengrößen zwischen 50 und 500 nm liegen.

Die Herstellung solcher Kolloide nach dem Stand der Technik ist dem Fachmann auf dem Gebiet hinreichend bekannt. In der Regel wird dabei ein Tetraalkylorthosilikat in einer alkoholischen Ammoniak-Phase dispergiert. Innerhalb kurzer Zeit werden durch Hydrolyse der Silane in der Dispersion sphärische Nanopartikel gebildet, deren Teilchengrößen von der Art der eingesetzten Reaktionspartner, deren Konzentration, dem Lösungsmittel, dem Verhältnis der Phasen untereinander und der Temperatur bestimmt werden. So sind in der Regel die Reaktionsraten in Methanol höher als in n-Butanol; entsprechend liefert die Reaktion in Methanol die kleinsten Teilchengrößen im Vergleich zu höheren Alkoholen. Der Einfluß der Alkoxysilane auf die Teilchengrößen ist allgemein bekannt, die Teilchengröße nimmt beim Übergang von Methylestern zu höhermolekularen Estern zu. In ähnlicher Weise lassen sich die Partikelgrößen auch durch Variation der Ammoniak-Konzentration beeinflussen: mit zunehmender Konzentration nimmt in der Regel die Teilchengröße ab.

Mit Hilfe dieses Verfahrens werden, je nach Reaktionsbedingungen, Teilchen mit einer Größe zwischen 50 und 500 nm selektiv gebildet.

Durch Zumischen der SiO₂-Kolloid zu den Silica-Solen werden die Silica-Kolloide bei der anschließenden Bead-Herstellung in Suspension überraschenderweise in der Weise in die Silica-Beads integriert, dass die entstehenden Partikel gegenüber dem Stand der Technik (PCT/EP01/08392) bekannten Silica-Partikeln eine um den Faktor 2 bis 5 größere zugängliche Oberfläche aufweisen. Die Konzentration der zugesetzten SiO₂-Kolloid beträgt in der Regel 10 bis 40 Vol %, vorzugsweise 20 bis 35 Vol.-%, bezogen auf die Hydrosol-Phase, wobei die SiO₂-Kolloide einen Feststoffanteil von 10 bis 50 Gew.-% enthalten.

Außer der Zugabe von SiO₂-Kolloide als Parameter zur Steigerung der Nukleinsäurebindung, hat sich überraschenderweise gezeigt, dass auch die Präsenz bestimmter Metalloxide oder Halbmetalloxide in den Silica-Trägern einen zusätzlichen positiven Effekt in Hinblick auf die NukleinsäureBindung besitzt. Als besonders effizient haben sich hierbei die Oxide der Metalle Titan, Kupfer, Kobalt, Aluminium, Kalzium, Zirkonium, Mangan, Kalium, Barium, Magnesium und/oder Zink sowie der Halbmetalle Bor und Arsen erwiesen, wobei diese Auswahl lediglich als Beispiel und nicht als Einschränkung der Erfindung anzusehen sind. Besonders bevorzugt im Sinne der Erfindung sind Boroxid (B₂O₃) und Zinkoxid. Zur Inkorporation der Metalloxide werden entsprechende metallorganische Verbindung z.B. in Form von Alkylderivaten, Alkoholaten, Acetaten oder Alkoxiden den Hydrosolen zugemischt, so dass die zugesetzte(n) Metallverbindung(en) oder Halbmetallverbindung(en) bei der Umwandlung von dem Hydrosol in das Silicagel als Oxid(e) in die Silicagel-Matrix inkorporiert wird/werden.

Unter dem Gesichtspunkt der zu verbessernden Nukleinsäurebindung im Vergleich zu den aus dem Stand der Technik bekannten Trägermedien haben sich vor allem Silica-Träger mit einem definierten Boroxid- und Zinkoxidgehalt als besonders gut geeignet herausgestellt. Dabei beträgt der Boroxid-Gehalt vorzugsweise 5 - 15 Mol % und der Zinkoxid-Gehalt 2 bis 10 Mol % (bezogen auf den Silica-Gehalt). Die Konzentrationen der übrigen Metalloxide liegen in der Regel im Bereich von 1 bis 20 Mol %. Durch die Inkorporation von insbesondere Bor- und Zinkoxiden konnte die NukleinsäureBindung um mehr als 25 % gegenüber Trägern nach dem Stand der Technik gesteigert werden. Die Integration der Metalloxide in die SiO₂-Matrix erfolgt im allgemeinen durch Zusammenmischen der entsprechenden organischen Komponenten mit dem gebildeten Hydrosol. Die korrespondierenden Oxide entstehen dann bei der nachfolgend beschriebenen Wärmebehandlung des Gels.

Über die oben beschriebenen Modifikationsschritte hinaus hat sich eine weitere Verfahrensweise als nukleinsäurebindungssteigernd herausgestellt. Dies betrifft eine Temperaturnachbehandlung der mit Hilfe der Dispersionsvernetzung gewonnenen sphärischen Silica-Partikel ("Beads"). Die nach dem Stand der Technik bekannten Gele (PCT/EP01/08392), liegen in der Regel als Hydrogele mit einem hohen Anteil gebundenen Wassers vor. Die daraus resultierenden hydrophilen Eigenschaften der Träger verhindern eine signifikante Nukleinsäurebindung (d.h. mehr als 1 mg/g Träger), so dass diese nur sehr eingeschränkt für routinemäßige Analysen herangezogen werden können. Dieser Nachteil läßt sich bei dem erfindungsgemäßen Verfahren nun überraschenderweise dadurch beheben, dass die gewonnen Hydrogele einer Temperaturbehandlung unterzogen werden, die das Wasser vollständig aus dem Träger entfernt und dadurch die Kieselgel-Partikel in feste, wasserfreie SiO₂-Träger, die auch allgemein als *Xerogele* oder *Silicagele* bezeichnet werden, umwandelt. Die Temperaturbehandlung erfolgt in der Regel oberhalb 250 °C, vorzugsweise oberhalb 500 °C, wobei vorzugsweise temperaturgeregelte Muffelöfen zur Anwendung gelangen. Die Temperaturbehandlung dauert in der Regel 1 bis 2 Stunden, je nach Größe und Wassergehalt der polymeren Träger.

Die Teilchengrößen der mittels inverser Dispersionsvernetzung hergestellten Polymerbeads lassen sich sowohl über die Viskosität der wäßrigen Polymerphase als auch über den mechanischen Rührprozess einstellen. So werden Teilchen mit einer Größe < 100 µm vorwiegend bei einer Viskosität des Sols < 40 cp und Teilchen > 200 µm aus Solen mit einer Viskosität > 40 cp gebildet.

Zur Herstellung besonders feiner Partikel-Fraktionen (< 10 µm) ist ein vorzugsweise handelsübliches Dispergierwerkzeug, das nach dem Rotor-Stator-Prinzip arbeitet (z.B. Ultra-Turrax®) und eine Umdrehungsleistung von > 10.000 U/min aufweist erforderlich. Größere Polymerbeads (> 20 µm) lassen sich demgegenüber mit herkömmlichen Rührern bei einer Rührgeschwindigkeit von 800 - 5000 U/min herstellen. Der Rührvorgang dauert in der Regel 3 bis 10 Sekunden. Die gewonnenen Magnetpartikel können anschließend mit Hilfe eines Handmagneten aus der Dispersion abgetrennt und durch Waschen mit Alkohol und Wasser gereinigt werden. Bevorzugt werden so Silicagel-Partikel erhalten, die Partikelgrößen zwischen 0,5 und 1 µm, 1 bis 10 µm, 10 bis 30 µm, 30 bis 100 µm und >100 µm aufweisen.

Die gewonnenen Gel-Partikel können im Anschluß an die oben beschriebene Temperaturbehandlung direkt gemäß den bekannten Methoden zur Aufreinigung von Nukleinsäuren verwendet werden. In Hinblick auf Techniken zur Nukleinsäure-Isolierung wird auf Sambrook et al.: Molecular Cloning, A Laboratory Manual, 2nd Edition, Cold Spring Harbour Lab. Press, Cold Spring Harbour, New York, verwiesen.

Um den Silica-Beads magnetische Eigenschaften zu verleihen, werden den Silica-Solen vor der Dispersion in der organischen Phase magnetische Substanzen zugemischt. Hierfür sind z.B. Magnetkolloide oder Ferrofluide sowie ferro-, ferri-oder superparamagnetische Mikro- oder Nanopartikel, die über ein solches magnetisches Moment verfügen, so dass nach ihrer Einkapselung die Silica-Beads mit einem herkömmlichen Handmagneten abgetrennt werden können. Die Kolloide bzw. Ferrofluide sind z.T. kommerziell verfügbar oder ihre Herstellung ist in der Literatur hinreichend beschrieben
(siehe z.B. PCT/EP96/02398 und darin zitierte Literatur) und kann von dem Fachmann auf diesem Gebiet jeder Zeit nachvollzogen werden.

Das entscheidende Kriterium für die Auswahl geeigneter Kolloide bzw. Ferrofluide stellt ihre homogene Dispergierbarkeit im Silica-Sol dar, d.h., das Magnetkolloid darf im Kontakt mit der Sol-Phase nicht ausflocken oder agglomerieren. Vor allem Ferrofluide, die geladene Tenside z.B. in Form aromatischer oder aliphatischer Sulfonsäurederivate oder aliphatischer Carbonsäuren zur Stabilisierung enthalten, sind dafür besonders geeignet. Derartige Magnetsubstanzen sind auch, wie schon erwähnt, kommerziell erhältlich.

Die Möglichkeit der einfachen und gezielten Einstellung des Magnetanteils im erfindungsgemäßen Silicagel-Partikel durch Zumischen des Magnetkolloids, die dieses Verfahren gegenüber dem Stand der Technik auszeichnet, eröffnet ein breites Anwendungsspektrum, das über die bloße Auftrennung von Biomolekülen und Nukleinsäuren oder die Biomolekülanalyse, wie in PCT/EP01/08392 und der darin zitierten Literatur beschrieben, weit hinausgeht.

Neben den in nanopartikulärer Form vorliegenden Magnetkolloiden oder Ferrofluiden sind grundsätzlich auch Magnetpartikel zur Einkapselung verwendbar, die über eine feste Polymerhülle verfügen. Solche Magnetbeads, die eine Hülle aus Polyvinylacetat, Polyvinylalkohol, Dextran, Polyacrolein, Polystyrol, Albumin oder Alginat aufweisen und allgemein Teilchengrößen von 0,05 bis 5 µm aufweisen, sind aus dem Stand der Technik bekannt (siehe z.B. PCT/EP96/02398 und darin zitierte Literatur) und werden auch kommerziell u.a. unter den Bezeichnungen Dynabeads, BioMag, Estapor, M-PVA, AGOWA, BioBeads oder SPHERO angeboten, wobei es sich z.T. um eingetragene Warenzeichen handelt.Diese Magnetpartikel werden in analoger Weise wie die Kolloide bzw. Ferrofluide zur Herstellung der erfindungsgemäßen Silica-Partikel eingesetzt.

Nach Zugabe der Magnetkolloide, der die Oberfläche vergrößernden SiO₂-Kolloide sowie der Metallverbindungen zu dem Silica-Sol erfolgt die Dispersion der Mischung in einem organischen Dispergiermittel. Als geeignete Dispergiermittel kommen dafür Lösungsmittel in Frage, die mit der Hydrosol-Phase nicht-mischbar sind und in dem die Hydrosol-Phase in der Lage ist stabile, definierte Tröpfchen zu bilden. Beispiele hierfür sind Hexan, Petrolether, Toluol, Tetrachlorkohlenstoff, Chloroform, Trichlorethylen, 1.1.1-Trichlorethan, Heptan oder Octan. Vorzugsweise kommen solche Lösungsmittel in Betracht, die einen Verteilungskoeffizienten (gemäß Definition nach C. Laane et al., "Biocatalysis in Organic Media", Laane et al. Hrsg., Elsevier, Amsterdam, pp 65, 1987) > 2 aufweisen. Auch Mischungen der obigen Lösungsmittel mit einer Dichte von ca. 1 g/cm³ sind gut zum Dispergieren geeignet.

Zur Steigerung der Qualität der Silica-Dispersionen im Hinblick auf Uniformität und Kugelgestalt hat es sich überraschenderweise als vorteilhaft erwiesen, der organischen Phase einen oder mehrere Emulgatoren bzw. Tenside zuzusetzen. Hierzu zählen oberflächenaktive Substanzen, bzw. Tenside und Stabilisatoren wie z.B.: Propylenoxid-Ethylenoxid-Blockcopolymere, Polyglycerinester, Polyoxyethylen-Sorbitan-Fettsäureester, Alkylphenylpolyethylenglykol-Derivate, Polyethylenglykol-Castoröl-Derivate, Blockcopolymere aus Rizinusöl-Derivaten, Polyethylenglykol-ether-derivate, Polyoxypropylen-Ethylendiamin-Blockcopolymere, Sorbitan-Fettsäureester, Polyethylenglykole, Polyoxyethylen-, modifizierte Polyester, Polyoxyethylen-Alkohol-Derivate, Polyhydroxyfettsäure-Polyethylenglykol-Blockcopolymere. Substanzen dieser Art sind kommerziell im Handel u.a. unter der Handelsbezeichnung: Synperonic^{®}, Arlacel^{®}, Brij^{®}, Renex^{®}, Estol^{®}, Eumulgin^{®}, Pluronic^{®}, Triton^{®}, Pripol^{®}, Hypermer^{®}, Span^{®}, Tween^{®}, Tetronic^{®}, Prisorine^{®}, Dehymuls^{®} oder Lameform^{®} erhältlich.

Die für die Herstellung der Magnetpartikel relevanten Emulgatorkonzentrationen liegen zwischen 0,1 und 15 Gew.-%, vorzugsweise zwischen 0,5 und 5 Gew.-%, bezogen auf das Dispergiermittel.

Alternativ zu klassischen organischen Lösungsmitteln als Dispergiermedium lassen sich auch herkömmliche Pflanzenöle mit einer Viskosität von 50 bis 500 cp sowie Mischungen von Pflanzenölen mit den organischen Lösungsmitteln einsetzen. Die Verwendung organischer Lösungsmittel hat jedoch gegenüber den Ölen den Vorteil, dass diese eine geringere Viskosität besitzen, wodurch die Separation der Silica-Partikel aus dem Reaktionsgemisch sowie die anschließenden Waschvorgänge innerhalb weniger Sekunden mit Hilfe eines handelsüblichen Handmagneten durchgeführt werden kann. Im Falle der Öle würde die Separation einschließlich der Waschprozesse erheblich länger (z.T. über Stunden) in Anspruch nehmen. Ein weiterer Vorteil ist die Möglichkeit, die organischen Flüssigkeiten durch Redestillation wiederzugewinnen.

Das Volumenverhältniß organische Phase zu Hydrosol beträgt in der Regel 5:1 bis 30:1, das Volumenverhältnis Hydrosol zu Magnetkolloid 2:1 bis 4:1 , wobei der Gewichtsanteil des magnetischen Festkörpers im Sol-Ansatz 15-50 % beträgt.

Im letzten Syntheseschritt werden die Magnetkolloide-, SiO₂-Kolloide und Metallverbindungen-enthaltenden Silica-Sole während des Dispersionsvorganges durch Zugabe einer Base zu definierten sphärischen Silica-Partikeln verfestigt. Die Basenzugabe führt innerhalb kurzer Zeit (bevorzugt 3 bis 20 Sekunden, i.a. weniger als 60 Sekunden) zu einer Verfestigung (Gelbildung) der Polymertröpfchen. Die Gelbildungsgeschwindigkeit ist dabei um so größer, je höher die Konzentration der eingesetzen Base ist. Als Basen kommen vorzugsweise Ammoniak oder NaOH zum Einsatz. Natronlauge wird in der Regel als 0,05 bis 0,1 molare Lösung, Ammoniak in Form einer 1 bis 12 %igen wäßrigen Lösung eingesetzt. Die Volumenverhältnisse Base zu Sol liegen üblicherweise bei 1:2 bis 1:4.

Da die Gelierungsreaktion sehr rasch abläuft, erfordert der Herstellungsprozeß für die Basispartikel einschließlich der Synthese des Sols und des Magnetkolloids einen Zeitaufwand von weniger als einer Stunde. Das bedeutet gegenüber allen herkömmlichen Verfahren mindestens eine Zeitersparnis von 30 bis 90 %.

Neben dem Einsatz der erfindungsgemäßen Silicagel-Beads mit vergrößerter Oberfläche speziell für die Nukleinsäureaufreinigung können die Silicagele darüber hinaus so modifiziert werden, dass ihr Einsatz in der Separationstechnologie signifikant erweitert werden kann. Es ist aus Patentschriften DE 32 11 309 (entsprechend US 4,699,717), hier als Referenz angeführt, bekannt, daß insbesondere Medien mit kationischen Gruppen (Anionenaustauscher) für die Nukleinsäure- und Protein-Auftrennung hervorragend geeignet sind. Ein solcher Trägertyp läßt sich durch chemische Umsetzung der Silicagel-Partikel mit Epoxysubstituiertem Alkoxysilan wie z.B. 3-Glycidyloxypropyltrimethoxysilan oder 3-Glycidyloxypropylmethyldiäthoxysilan und anschließende nukleophile Öffnung des Oxiranringes mittels tertiärer oder sekundärer Alkylamine herstellen. Auch die Synthese stark und schwach saurer Ionenaustauscher sowie von Metallchelat-Trägern ist durch Umsetzung der beschriebenen Epoxy-substituierten Silicagel-Partikel mit Hilfe von Carbonsäuren, Sulfiten, Thiosulfaten bzw. Aminosubstituierten Carbonsäuren, z.B. Nitrilotriessigsäure oder Iminodiessigsäure, möglich.

Die Funktionalisierung der Silica-Basisbeads zu speziellen Trägersystemen ist nicht nur auf die Synthese von von Ionenaustauschern beschränkt. In einer besonderen Ausführungsform können die SiO₂-Träger mit substituierten Alkylalkoxysilanen der allgemeinen Formel X-(CH₂)ₙ-Si-(OR)₃, wobei X ein Halogen, Cyano-, NH₂- oder Mercapto-Rest, n = 1-6, vorzugsweise 3, R ein Alkyl-, Trialkysilyl-Rest oder H ist, umgesetzt werden. An die so modifizierten Träger lassen sich, sei es für die Separation nach dem Affinitätsprinzip oder für den Einsatz als Biokataylsatoren, Liganden in Form von Peptiden, Proteinen oder Enzymen kovalent binden. Proteine und andere Liganden können dabei durch einfache Inkubation mit den Halogen-substituierten Trägern direkt gekoppelt werden.

Ohne auf weitere detaillierte Ausführungen diese Kopplungen und Modifikationen betreffend einzugehen, die u.a. in "Methods in Enzymology", Vol. 135, Part B, Hrsg. K. Mosbach, Academic Press, Orlando, 1987, in "Scientific and Clinical Applications of Magnetic Carriers", Häfeli et al. (Hrsg.), Plenum Press, New York ,1997, sowie in "Immobilized Biomolecules in Analysis", T. Cass und F.S. Ligler Hrsg., Oxford University Press, 1998, beschrieben sind, wird davon ausgegangen, daß ein Fachmann auf diesem Gebiet die speziellen Reaktionsmethoden hinreichend kennt und daher die Beschreibung grundsätzlich nutzen kann. Die beschriebenen Ausführungsformen sind daher in keiner Weise als limitierende Offenbarungen aufzufassen.

In den nachfolgenden Beispielen werden die erfindungsgemäßen Verfahren und Produkte näher beschrieben.

### Beispiel 1

Präparation A: 5 ml Tetraethoxysilan werden mit 37 ml Propanol vermischt und rasch mit einer Lösung, bestehend aus 1,9 ml 25 %igem Ammoniak und 3,7 ml Wasser, verrührt. Nach 30 Minuten fallen SiO₂-Kolloid mit einer mittleren Teilchengröße von 223 nm an. Das Kolloid wird anschließend abzentrifugiert und der Überstand abpipettiert. Das Präzipitat wird 5 Minuten im Ölpumpenvakuum getrocknet, in 5 ml Wasser aufgenommen und unter Anwendungen eines Ultraschallbades redispergiert.

Präparation B: Eine Mischung aus 90 ml Tetraethoxylsilan, 10 ml Wasser und 8,5 ml 0,1 M HCl werden in einem Ultraschallbad solange beschallt (ca. 20 Minuten) bis sich eine homogene Mischung bildet.

Der Präparation B werden sodann 25 ml eines Magnetkolloides, das gemäß der Vorschrift von Shinkai et al. (Biocatalysis, Vol 5, 61, 1991) durch Oxidation einer 0,6 molaren Eisen(II)salz-Lösung, die unter Verwendung von 0,3 M Na-Nitrit hergestellt wurde, zugegeben. Es folgt eine kurze Beschallung im Ultraschallbad für 2 Minuten. Die erhaltene Magnetdispersion wird sodann mit 5 ml der Präparation A und 6,5 g Zink-(2,2,6,6-tetramethyl-3,5-heptandionat) versetzt und nochmal 2 Minuten beschallt. Die erhaltene Dispersion wird in einem Liter Trichlorethylen, in dem 2,5 Gew.-% Brij 52 und 1,8 Gew.-% Tween 85 gelöst sind, eingetragen. Die Dispersion wird unter Rühren (1500 U/Min.) einige Sekunden dispergiert und sodann mit 45 ml 1 %iger Ammoniak-Lösung versetzt. Es wird 5 Sekunden weitergerührt. Nach 5 Minuten werden die Magnetpartikel mit Hilfe eines handelsüblichen Handmagneten aus der Dispersion abgetrennt und je fünfmal mit ca. 50 ml Methanol und Wasser nachgewaschen. Es werden Magnetpartikel mit einer mittleren Teilchengröße von 38 µm gewonnen. Nach 12-stündiger Inkubation in Wasser werden die Partikel nochmals mehrfach mit Wasser gewaschen und anschließend ca. eine Stunden im Vakuum getrocknet. Anschließend werden die Partikel in einem Muffelofen bei 650 °C 1 Stunde erhitzt. Die so gewonnen Magnetpartikel können nach den bekannten Methoden zur Aufreinigung von Nukleinsäuren verwendet werden.

### Beispiel 2

Präparation A: Ein SiO₂-Kolloid wirdaus einer Mischung, bestehend aus 0,63 ml Wasser, 2,35 ml gesättigter wäßriger Ammoniaklösung, 0,3 ml Tetraethoxysilan und 1,69 ml Ethanol, hergestellt. Es fallen Teilchen mit einer mittleren Größe von 245 nm an. Die weitere Vakuumbehandlung sowie Aufarbeitung und Redispersion des Kolloids erfolgt analog Beispiel 1.

Präparation B: Eine Mischung, bestehend aus 100 ml Tetraethoxysilan, 20 ml Wasser und 5 ml 0,05 M HCl, wird unter Zuhilfenahme eines Ultraschallbades homogenisiert.

Zu dieser Sol-Phase werden 30 ml Magnetkolloid (analog Beispiel 1) zugemischt und 2 Minuten im Ultraschallbad behandelt. Diese Dispersion wird anschließend mit der Präparation A sowie mit 4,8 g Zinkacetat vermischt und für 5 Minuten beschallt.

Die erhaltene Dispersion wird in 3 Litern Trichlorethylen, das 3,5 Gew.-% Span 60 und 1,5 Gew.-% Tween 85 gelöst enthält, eingetragen und unter Rühren (2500 U/Min.) 4 Sekunden dispergiert. Es folgt die unmittelbare Zugabe von 55 ml 1 %iger Ammoniaklösung. Es wird 5 Sekunden weitergerührt. Nach 5 Minuten werden die Magnetpartikel mit Hilfe eines handelsüblichen Handmagneten aus der Dispersion abgetrennt und je fünfmal mit ca. 50 ml Methanol und Wasser nachgewaschen. Es werden Magnetpartikel mit einer mittleren Teilchengröße von 24 µm gewonnen. Nach 12-stündiger Inkubation in Wasser werden die Partikel nochmals mehrfach mit Wasser gewaschen und anschließend ca. eine Stunden im Vakuum getrocknet. Anschließend werden die Partikel in einem Muffelofen bei 650°C 2 Stunden erhitzt. Die so gewonnen Magnetpartikel können nach den bekannten Methoden zur Nukleinsäureisolierung aus biologischen Flüssigkeiten eingesetzt werden.

### Beispiel 3

25 ml Tetraethoxysilan werden mit 7,5 ml Wasser und 2,5 ml 0,15 M HCl versetzt und analog Beispiel 1 homogenisiert. Dieser Sol-Phase werden 12 ml Ferrofluid EMG 507 (Fa. FerroTec, Nashua, USA), 4,5 ml Triethylborat, 2,8 ml SiO₂-Kolloid, das analog Beispiel 1 synthetisiert wurde, und 0,8 g Zink-(2,2,6,6-tetramethyl-3,5-heptandionat) zugesetzt. Die Mischung wird für 5 Minuten unter Eiskühlung im Ultraschallbad beschallt. Die Dispersion wird anschließend unter Rühren (1800 U/min) in 450 ml Hexan, das 1,5 Gew.-% Span 80 und 4,5 Gew.-% Dehymuls HRE gelöst enthält, dispergiert. Während des Dispergiervorganges werden 12 ml 1 %ige Ammoniaklösung zugefügt. Es wird 5 Sekunden weitergerührt. Separation und Aufarbeitung der gewonnenen Magnetpartikel erfolgt analog Beispiel 1. Es entstehen Träger mit einer mittleren Teilchengröße von 84 µm. Die abgetrennte Magnetpartikel-Fraktion wird analog Beispiele 1 mit Methanol und Wasser gewaschen. Es folgt mehrfaches Waschen mit je 30 ml getrocknetem Toluol, dem sich eine zweistündige Vakuumtrocknung anschließt. Das anfallende Produkt wird sodann 3 Stunden bei 120 °C und anschließend nochmal 1 Stunden bei 650 °C im Muffelofen erhitzt. Das Produkt wird anschließend 12 Std. nach Zugabe von 25 ml über Molekularsieb getrocknetem Toluol und 0,5 g 3-Aminopropyltriethoxysilan am Rückfluß erhitzt. Die Magnetpartikel werden wieder magnetisch abgetrennt und je fünfmal mit Toluol und Chloroform nachgewaschen. Es folgt mehrstündige Trocknung im Vakuum. Das aminomodifizierte Produkt wird anschließend mit 6 %iger Glutaraldehyd-Lösung in 10 ml 0,1 M Na-Carbonat-Puffer, pH 9.2, für 3 Stunden bei 35°C umgesetzt. Es wird anschließend intensiv mit 0,1 M Phosphat-Puffer, pH 7.2, nachgewaschen. Die gewonnenen Aldehyd-funktionalisierten Magnetpartikel werden in 8,5 ml 0,1 M Phosphat-Puffer suspendiert und in 2 ml 0,1 M Phosphat-Puffer, pH 7.2, in dem 5,5 mg Streptavidin gelöst sind, inkubiert. Nach sechsstündiger Reaktion bei 40 °C wird das Produkt fünfmal mit Phosphat-Puffer nachgewaschen. Um verbliebene Rest-Aldehydgruppen abzusättigen, wird das magnetisch abgetrennte Produkt in 10 ml 0,2 M Äthanolamin bei Raumtemperatur über einen Zeitraum von 5 Stunden inkubiert. Die anschließend mehrfach mit Phosphat-Puffer gewaschenen Magnetpartikel können direkt nach den bekannten Verfahren zur Bindung biotinylierter Nukleinsäuren oder biotinylierter Proteine verwendet werden.

## Patentansprüche

1. Sphärische Silicagel-Partikel, die einen Gehalt an Magnetpartikeln aufweisen, und in die SiO₂-Kolloide und ein oder mehrere Oxide, die aus der Gruppe ausgewählt sind, die Metalloxide und Halbmetalloxide umfaßt, eingekapselt sind.

2. Silicagel-Partikel gemäß Anspruch 1, **dadurch gekennzeichnet, dass** der Anteil der SiO₂-Kolloide im Partikel 10 bis 40 Gew.-% beträgt, wobei die eingelagerten SiO₂-Kolloide vorzugsweise eine Teilchengröße von 50 bis 500 nm aufweisen, und der Anteil der Oxide vorzugsweise 1 bis 20 Mol %, bezogen auf den Silica-Gel-Gehalt, beträgt.

3. Silicagel-Partikel gemäß Anspruch 1 oder 2, **dadurch gekennzeichnet, dass** die Partikel vorzugsweise bei Temperaturen oberhalb 250°C temperaturbehandelt sind.

4. Silicagel-Partikel gemäß Anspruch 1 bis 3, **dadurch gekennzeichnet, dass** der Magnetpartikelgehalt in den Silica-gel-Partikeln 15 bis 50 Gew.-% beträgt, wobei die Magnetpartikel vorzugsweise aus der Gruppe ausgewählt sind, die Magnetkolloide, Ferrofluide und ferro-, ferri- oder superparamagnetische Nanopartikel umfaßt.

5. Silicagel-Partikel gemäß einem der Ansprüche 1 bis 4, **dadurch gekennzeichnet, dass** die Partikelgrößen zwischen 0,5 und 1 µm, 1 bis 10 µm, 10 bis 30 µm, 30 bis 100 µm oder > 100 µm eingestellt sind.

6. Silicagel-Partikel gemäß einem der Ansprüche 1 bis 5, **dadurch gekennzeichnet, dass** die Oxide ausgewählt sind aus der Gruppe, die Zirkon, Titan, Kupfer, Kobalt, Aluminium, Kalzium, Kalium, Mangan, Barium, Magnesium und Zink umfasst, und die Halbmetalloxide ausgewählt sind aus der Gruppe, die Bor und Arsen umfasst.

7. Silicagel-Partikel gemäß einem der Ansprüche 1 bis 6, **dadurch gekennzeichnet, dass** die Partikel funktionelle Gruppen aufweisen, die mit Verbindungen reagieren, ausgewählt aus der Gruppe der Oligopeptide, Proteine, Oligosaccharide, Polysaccharide, Nukleinsäuren, Antikörper, Antikörperfragmente Enzyme oder Kombinationen daraus, und wobei die funktionellen Gruppen vorzugsweise aus der Gruppe ausgewählt sind, die Aldehyd-, Carboxyl-, Amino-, Hydroxyl-, Mercapto- und Oxiranfunktionen oder Kombinationen daraus umfasst.

8. Verfahren zur Herstellung sphärischer, magnetischer Silicagel-Partikel, **dadurch gekennzeichnet dass**:
a) Alkoxysilane durch saure Katalyse zu einem Hydrosol umgewandelt werden,
b) dem Hydrosol ein SiO₂-Kolloid zur Vergrößerung der Oberfläche der Silicagel-Partikel zugemischt wird,
c) dem Hydrosol magnetische Partikel zugesetzt werden, so daß die Silicagel-Partikel magnetische Eigenschaften erhalten,
d) dem Hydrosol eine oder mehrere Verbindungen, ausgewählt aus der Gruppe der Metallverbindungen oder Halbmetallverbindungen oder Kombinationen daraus, zugesetzt wird/werden,
e) die so erhaltene Hydrosol-Mischung, die die Magnetpartikel, Verbindungen, ausgewählt aus der Gruppe der Metallverbindungen oder Halbmetallverbindungen oder Kombinationen daraus, und SiO₂-Kolloide enthält, in einem mit der Hydrosol-Phase nicht mischbaren Dispergiermittel dispergiert wird,
f) die Hydrosol-Mischung, die die Magnetpartikel, Metallverbindung(en) und SiO₂-Kolloide enthält, während des Dispergiervorganges durch Basenzugabe zu festen Gel-Partikeln verfestigt wird, und
g) die so erhaltenen Gel-Partikel anschließend durch eine Temperaturbehandlung zu festen Silicagel-Partikeln (Xerogelen) umgewandelt werden.

9. Verfahren nach Anspruch 8, **dadurch gekennzeichnet, dass** das Volumenverhältniß von Hydrosol zu Dispergiermittel 1:5 bis 1:30 beträgt, und dass vorzugsweise als Dispergiermittel Pflanzenöle mit einer Viskosität von 50 bis 500 cp oder organische Lösungsmittel mit einem Verteilungskoeffizienten > 2 im Oktanol-Wasser-System, die aus der Gruppe ausgewählt sind, die chlorierte Kohlenwasserstoffe, Alkane mit einer Kohlenstoffkettenlänge mit mehr als 7 C-Atomen oder Aromaten oder Mischungen derselben umfasst, verwendet werden.

10. Verfahren nach einem der Ansprüche 8 und 9, **dadurch gekennzeichnet, dass** die Dispergierung mechanisch mit einer Rührgeschwindigkeit von 500 bis 20.000 U/Min. und nicht länger als 10 Sekunden gerührt wird, so dass vorzugsweise ein vorbestimmter Teilchendurchmesser im Bereich von 0,5 bis 2000 µm erzielt wird, wobei Teilchendurchmesser zwischen 0,5 und 1 µm, 1 bis 10 µm, 10 bis 30 µm, 30 bis 100 µm oder > 100 µm besonders bevorzugt sind.

11. Verfahren nach einem der Ansprüche 8 bis 10, **dadurch gekennzeichnet, dass** als Alkoxysilane Kieselsäureorthoester aliphatischer Alkohole mit einer Kohlenstoffkettenlänge von 1 bis 5 C-Atomen verwendet werden.

12. Verfahren nach einem der Ansprüche 8 bis 11, **dadurch gekennzeichnet, dass** bei der säurekatalysierten Hydrolyse der Alkoxysilane die Säure ausgewählt ist aus der Gruppe, die verdünnte Mineralsäuren, Organische Säuren oder Kombinationen daraus umfasst, wobei der Volumenanteil der verdünnten Mineralsäuren in der Hydrosol-Silan-Phase 10 bis 35 % und vorzugsweise 20 bis 28 % beträgt, und der Volumenanteil der organischen Säuren in der Hydrosol-Phase vorzugsweise 15 bis 40 % beträgt.

13. Verfahren nach einem der Ansprüche 8 bis 12, **dadurch gekennzeichnet, dass** die Umwandlung der Alkoxysilane zum Hydrosol für 5 bis 30 Minuten unter Ultraschall durchgeführt wird.

14. Verfahren nach einem der Ansprüche 8 bis 13, **dadurch gekennzeichnet, dass** die dem Hydrosol zugesetzten magnetischen Substanzen ausgewählt sind aus der Gruppe, die Magnetkolloide, Ferrofluide, ferro-, ferri- oder superparamagnetische Partikel umfasst.

15. Verfahren nach einem der Ansprüche 8 bis 14, **dadurch gekennzeichnet, dass** dem Dispergiermittel eine oder mehrere oberflächenaktiven Substanz(en) zugemischt werden, ausgewählt vorzugsweise aus der Gruppe, die Sorbitan-Fettsäureester, Propylenoxid-Ethylenoxid-BLockcopolymere, Polyglycerinester, Polyoxyethylen-Sorbitan- Fettsäureester, Alkylphenylpolyethylenglykol-Derivate, Polyethylenglykol-Castoröl-Derivate, Blockcopolymere aus Rizinusöl-Derivaten, Polyethylenglykol-Etherderivate, Polyoxypropylen-Ethylendiamin-Blockcopolymere, Polyethylenglykole, Polyoxyethylen-, modifizierte Polyester, Polyoxyethylen-Alkohol-Derivate und/oder Polyhydroxyfettsäure-Polyethylenglykol-Blockcopolymere oder Kombinationen daraus Umfaßt, wobei der der Anteil der Oberflächenaktivensubstanz(en) 0,1 bis 15 Gew.-%, bezogen auf das Dispergiermittel, beträgt.

16. Verfahren nach einem der Ansprüche 8 bis 15, **dadurch gekennzeichnet, dass** während des Dispergiervorgangs 10 bis 30 Vol.-%, bezogen auf die Hydrosol-Phase, einer 1 bis 20 %igen Base zugesetzt werden, wobei die Base vorzugsweise Ammoniak ist.

17. Verfahren nach einem der Ansprüche 8 bis 16, **dadurch gekennzeichnet, dass** der Anteil einer oder mehrerer Verbindungen im Hydrosol, die aus der Gruppe ausgewählt ist/sind, die Metallverbindungen, Halbmetallverbindungen oder Kombinationen daraus umfasst, 1 bis 20 Mol % beträgt, wobei die Metalle der Verbindung(en) vorzugsweise ausgewählt sind aus der Gruppe, die Zirkon, Titan, Kupfer, Kobalt, Aluminium, Kalzium, Kalium, Mangan, Barium, Magnesium und Zink umfasst, und wobei die Halbmetalle der Verbindung(en) vorzugsweise ausgewählt sind aus der Gruppe, die Bor und Arsen umfasst, und die Metallverbindungen vorzugsweise in Form von Verbindungen vorliegen, die ausgewählt sind aus der Gruppe, die Acetate, Fumarate, Alkoholate, Ethylendiamintetraessigsäure, Nitrilotriessigsäure, Alkylderivate, Porphinderivate und Carboxylatderivate oder Kombinationen daraus umfasst.

18. Verfahren nach den Ansprüchen 8 bis 17, **dadurch gekennzeichnet, dass** die Hydrosol-Partikel über einen Zeitraum von einer bis mehreren Stunden einer Temperaturbehandlung bei Temperaturen > 250 °C unterworfen und **dadurch** zu Silicagel-Partikeln umgewandelt werden.

19. Verfahren nach einem der Ansprüche 8 bis 18, **dadurch gekennzeichnet, dass** die Silicagel-Partikel in einem weiteren Verfahrensschritt umgesetzt werden mit substituierten Alkylalkoxysilanen der allgemeinen Formel X-(CH₂)ₙ-Si-(OR)₃, wobei X ausgewählt ist aus der Gruppe, die Aldehyd-, Epoxy, Halogen-, Cyano-, NH₂- oder Mercapto-Reste umfaßt, n = 1-6, vorzugsweise 3, und R einen Alkyl-, Trialkysilyl-Rest oder H bedeuten.

20. Verwendung der magnetischen Partikel nach einem der Ansprüche 1 bis 7 zur Aufreinigung oder Isolierung von Verbindungen ausgewählt aus der Gruppe, die Nukleinsäuren, Proteine, Antikörper, oder Antikörper-Fragmente sowie biotinylierter Nukleinsäure, Proteine, Antikörper oder Antikörper-Fragmente umfasst, als Ionenaustauscher oder Metallchelat-Träger, als Träger für Biokatalysatoren wie Proteine, Peptide, Enzyme, als Protein- und Zell-Einkapselungsmatrix oder als Adsorber für die Hemoperfusion.

## Claims

1. Spherical silica gel particles which have a content of magnetic particles and in which SiO₂ colloids and one or more oxides which are selected from the group including metal oxides and metalloid oxides are encapsulated.

2. Silica gel particles according to Claim 1, **characterized in that** the content of SiO₂ colloids in the particle is from 10 to 40% by weight, where the incorporated SiO₂ colloids preferably have a particle size of from 50 to 500 nm, and the content of oxides is preferably from 1 to 20 mol% based on the silica gel content.

3. Silica gel particles according to Claim 1 or 2, **characterized in that** the particles are thermally treated preferably at temperatures above 250°C.

4. Silica gel particles according to Claim 1 to 3, **characterized in that** the magnetic particle content in the silica gel particles is from 15 to 50% by weight, where the magnetic particles are preferably selected from the group including magnetic colloids, ferrofluids and ferromagnetic, ferrimagnetic or superparamagnetic nanoparticles.

5. Silica gel particles according to any of Claims 1 to 4, **characterized in that** the particle sizes are adjusted to between 0.5 and 1 µm, 1 to 10 µm, 10 to 30 µm, 30 to 100 µm or > 100 µm.

6. Silica gel particles according to any of Claims 1 to 5, **characterized in that** the oxides are selected from the group including zircon, titanium, copper, cobalt, aluminum, calcium, potassium, manganese, barium, magnesium and zinc, and the metalloid oxides are selected from the group including boron and arsenic.

7. Silica gel particles according to any of Claims 1 to 6, **characterized in that** the particles have functional groups which react with compounds selected from the group of oligopeptides, proteins, oligosaccharides, polysaccharides, nucleic acids, antibodies, antibody fragments, enzymes or combinations thereof, and where the functional groups are preferably selected from the group including aldehyde, carboxyl, amino, hydroxyl, mercapto and oxirane functions or combinations thereof.

8. Process for producing spherical magnetic silica gel particles, **characterized in that**:
a) alkoxysilanes are converted by acid catalysis into a hydrosol,
b) an SiO₂ colloid is admixed with the hydrosol to increase the surface area of the silica gel particles,
c) magnetic particles are added to the hydrosol so that the silica gel particles acquire magnetic properties,
d) one or more compounds selected from the group of metal compounds or metalloid compounds or combinations thereof is/are added to the hydrosol,
e) the hydrosol mixture obtained in this way, which comprises the magnetic particles, compounds selected from the group of metal compounds or metalloid compounds or combinations thereof, and SiO₂ colloids is dispersed in a dispersant which is immiscible with the hydrosol phase,
f) the hydrosol mixture which comprises the magnetic particles, metal compound(s) and SiO₂ colloids is consolidated to solid gel particles during the dispersion process by addition of base, and
g) the gel particles obtained in this way are subsequently converted by a thermal treatment into solid silica gel particles (xerogels).

9. Process according to Claim 8, **characterized in that** the ratio of hydrosol to dispersant is from 1:5 to 1:30 by volume, and **in that** vegetable oils with a viscosity of from 50 to 500 cp or organic solvents with a partition coefficient of > 2 in the octanol-water system, which are selected from the group including chlorinated hydrocarbons, alkanes with a carbon chain length of more than 7 C atoms or aromatic compounds or mixtures thereof are preferably used as dispersants.

10. Process according to either of Claims 8 and 9, **characterized in that** the dispersion is agitated mechanically with a stirring speed of from 500 to 20 000 rpm and for not longer than 10 seconds so that preferably a predetermined particle diameter in the range from 0.5 to 2000 µm is attained, with particle diameters of between 0.5 and 1 µm, 1 to 10 µm, 10 to 30 µm, 30 to 100 µm or > 100 µm being particularly preferred.

11. Process according to any of Claims 8 to 10, **characterized in that** orthoesters of silicic acid with aliphatic alcohols having a carbon chain length of from 1 to 5 C atoms are used as alkoxysilanes.

12. Process according to any of Claims 8 to 11, **characterized in that** the acid in the acid-catalyzed hydrolysis of the alkoxysilanes is selected from the group including dilute mineral acids, organic acids or combinations thereof, where the content of the dilute mineral acids in the hydrosol silane phase is from 10 to 35% by volume and preferably 20 to 28% by volume, and the content of the organic acids in the hydrosol phase is preferably from 15 to 40% by volume.

13. Process according to any of Claims 8 to 12, **characterized in that** the conversion of the alkoxysilanes into the hydrosol is carried out with ultrasound for from 5 to 30 minutes.

14. Process according to any of Claims 8 to 13, **characterized in that** the magnetic substances added to the hydrosol are selected from the group including magnetic colloids, ferrofluids, ferromagnetic, ferrimagnetic or superparamagnetic particles.

15. Process according to any of Claims 8 to 14, **characterized in that** one or more surface-active substance(s) selected preferably from the group including sorbitan fatty acid esters, propylene oxide-ethylene oxide block copolymers, polyglycerol esters, polyoxyethylene sorbitan fatty acid esters, alkylphenyl polyethylene glycol derivatives, polyethylene glycolcastor oil derivatives, block copolymers of castor oil derivatives, polyethylene glycol ether derivatives, polyoxypropylene-ethylenediamine block copolymers, polyethylene glycols, polyoxyethylene-, modified polyesters, polyoxyethylene alcohol derivatives and/or polyhydroxy fatty acid-polyethylene glycol block copolymers or combinations thereof, where the content of surface-active substance(s) is from 0.1 to 15% by weight based on the dispersant, are admixed with the dispersant.

16. Process according to any of Claims 8 to 15, **characterized in that** from 10 to 30% by volume based on the hydrosol phase of a 1 to 20% strength base are added during the dispersion process, the base preferably being ammonia.

17. Process according to any of Claims 8 to 16, **characterized in that** the content of one or more compounds which is/are selected from the group including metal compounds, metalloid compounds or combinations thereof in the hydrosol is from 1 to 20 mol%, where the metals of the compound(s) are preferably selected from the group including zircon, titanium, copper, cobalt, aluminum, calcium, potassium, manganese, barium, magnesium and zinc, and where the metalloids of the compound(s) are preferably selected from the group including boron and arsenic, and the metal compounds are preferably in the form of compounds which are selected from the group including acetates, fumarates, alcoholates, ethylenediaminetetraacetic acid, nitrilotriacetic acid, alkyl derivatives, porphine derivatives and carboxylate derivatives or combinations thereof.

18. Process according to Claims 8 to 17, **characterized in that** the hydrosol particles are subjected to a thermal treatment at temperatures > 250°C over a period of from 1 to several hours, and thus are converted into silica gel particles.

19. Process according to any of Claims 8 to 18, **characterized in that** the silica gel particles are reacted in a further process step with substituted alkylalkoxysilanes of the general formula X-(CH₂)ₙ-Si-(OR)₃, where X is selected from the group including aldehyde, epoxy, halogen, cyano, NH₂ or mercapto radicals, n = 1-6, preferably 3, and R is an alkyl, trialkylsilyl radical or H.

20. Use of the magnetic particles according to any of Claims 1 to 7 for purifying or isolating compounds selected from the group including nucleic acids, proteins, antibodies, or antibody fragments, and biotinylated nucleic acids, proteins, antibodies or antibody fragments, as ion exchangers or metal chelate supports, as supports for biocatalysts such as proteins, peptides, enzymes, as protein and cell encapsulation matrix or as adsorbent for hemoperfusion.

## Revendications

1. Particules sphériques de silicagel, qui présentent une teneur en particules magnétiques et dans lesquelles sont encapsulés des colloïdes de SiO₂ et un ou plusieurs oxydes, qui sont choisis dans le groupe comprenant les oxydes métalliques et les oxydes semi-métalliques.

2. Particules de silicagel selon la revendication 1, **caractérisées en ce que** la proportion de colloïdes de SiO₂ dans les particules est de 10 à 40% en poids, les colloïdes de SiO₂ incorporés présentant de préférence une grosseur de particules de 50 à 500 nm, et la proportion des oxydes étant de préférence de 1 à 20% en mole, par rapport à la teneur en gel silicique.

3. Particules de silicagel selon la revendication 1 ou 2, **caractérisées en ce que** les particules sont traitées thermiquement de préférence à des températures supérieures à 250°C.

4. Particules de silicagel selon les revendications 1 à 3, **caractérisées en ce que** la teneur en particules magnétiques dans les particules de silicagel est de 15 à 50% en poids, les particules magnétiques étant de préférence choisies dans le groupe comprenant les colloïdes magnétiques, les ferrofluides et les nanoparticules ferromagnétiques, ferrimagnétiques ou superparamagnétiques.

5. Particules de silicagel selon l'une quelconque des revendications 1 à 4, **caractérisées en ce que** les grosseurs des particules sont réglées entre 0,5 et 1 µm, 1 à 10 µm, 10 à 30 µm, 30 à 100 µm ou > 100 µm.

6. Particules de silicagel selon l'une quelconque des revendications 1 à 5, **caractérisées en ce que** les oxydes sont choisis dans le groupe comprenant le zirconium, le titane, le cuivre, le cobalt, l'aluminium, le calcium, le potassium, le manganèse, le baryum, le magnésium et le zinc et les oxydes semi-métalliques sont choisis dans le groupe comprenant le bore et l'arsenic.

7. Particules de silicagel selon l'une quelconque des revendications 1 à 6, **caractérisées en ce que** les particules présentent des groupes fonctionnels qui réagissent avec des composés choisis dans le groupe des oligopeptides, des protéines, des oligosaccharides, des polysaccharides, des acides nucléiques, des anticorps, des fragments d'anticorps, des enzymes ou leurs combinaisons et les groupes fonctionnels étant de préférence choisis dans le groupe comprenant les fonctions aldéhyde, carboxyle, amino, hydroxyle, mercapto et oxirane ou leurs combinaisons.

8. Procédé pour la préparation de particules sphériques, magnétiques de silicagel, **caractérisé en ce que**
a) des alcoxysilanes sont transformés par catalyse acide en un hydrosol,
b) l'hydrosol est mélangé avec un colloïde de SiO₂ en vue de l'agrandissement de la surface des particules de silicagel,
c) des particules magnétiques sont ajoutées à l'hydrosol, de manière telle que les particules de silicagel obtiennent des propriétés magnétiques,
d) un ou plusieurs composés, choisis dans le groupe des composés métalliques ou semi-métalliques ou leurs combinaisons est/sont ajouté(s) à l'hydrosol,
e) le mélange d'hydrosol ainsi obtenu, qui contient les particules magnétiques, des composés choisis dans le groupe des composés métalliques ou semi-métalliques ou leurs combinaisons et des colloïdes de SiO₂, est dispersé dans un dispersant non miscible avec la phase d'hydrosol,
f) le mélange d'hydrosol, qui contient les particules magnétiques, le/les composé(s) métallique(s) et les colloïdes de SiO₂ est solidifié pendant le processus de dispersion par addition de bases en particules de gel solides, et
g) les particules de gel ainsi obtenues sont ensuite transformées par un traitement thermique en particules solides de silicagel (xérogels).

9. Procédé selon la revendication 8, **caractérisé en ce que** le rapport volumique de l'hydrosol au dispersant est de 1:5 à 1:30, et **en ce qu'**on utilise de préférence comme dispersant des huiles végétales présentant une viscosité de 50 à 500 cp ou des solvants organiques avec un coefficient de répartition > 2 dans le système octanol-eau, qui sont choisis dans le groupe qui comprend les hydrocarbures chlorés, les alcanes avec une longueur de chaîne carbonée comprenant plus de 7 atomes de carbone ou des aromatiques ou des mélanges de ceux-ci.

10. Procédé selon l'une quelconque des revendications 8 et 9, **caractérisé en ce que** la dispersion est agitée mécaniquement à une vitesse d'agitation de 500 à 20 000 t/min et en n'agitant pas plus de 10 secondes, de manière à obtenir un diamètre de particules prédéterminé dans la plage de 0,5 à 2000 µm, des diamètres des particules entre 0,5 et 1 µm, 1 à 10 µm, 10 à 30 µm, 30 à 100 µm ou > 100 µm étant particulièrement préférés.

11. Procédé selon l'une quelconque des revendications 8 à 10, **caractérisé en ce qu'**on utilise comme alcoxysilanes des orthoesters d'acide silicique d'alcools aliphatiques avec une longueur de chaîne carbonée de 1 à 5 atomes de carbone.

12. Procédé selon l'une quelconque des revendications 8 à 11, **caractérisé en ce que** lors de l'hydrolyse catalysée par un acide des alcoxysilanes, l'acide est choisi dans le groupe comprenant des acides minéraux dilués, des acides organiques ou leurs combinaisons, où la proportion volumique des acides minéraux dilués dans la phase hydrosol-silane est de 10 à 35% et de préférence de 20 à 28%, et la proportion volumique des acides organiques dans la phase d'hydrosol est de préférence de 15 à 40%.

13. Procédé selon l'une quelconque des revendications 8 à 12, **caractérisé en ce que** la transformation des alcoxysilanes en hydrosol est réalisée pendant 5 à 30 minutes sous ultrasons.

14. Procédé selon l'une quelconque des revendications 8 à 13, **caractérisé en ce que** les substances magnétiques ajoutées à l'hydrosol sont choisies dans le groupe comprenant des colloïdes magnétiques, des ferrofluides, des particules ferromagnétiques, ferrimagnétiques ou superparamagnétiques.

15. Procédé selon l'une quelconque des revendications 8 à 14, **caractérisé en ce que** le dispersant est mélangé avec une ou plusieurs substance(s) tensioactive(s), de préférence choisies dans le groupe comprenant les esters de sorbitane d'acide gras, les copolymères à blocs d'oxyde de propylène-oxyde d'éthylène, les esters de polyglycérol, les esters d'acide gras de polyoxyéthylène-sorbitane, les dérivés d'alkylphénylpolyéthylèneglycol, les dérivés de polyéthylèneglycol-huile de ricin, les copolymères à blocs de dérivés d'huile de ricin, les dérivés de polyéthylèneglycoléther, les copolymères à blocs de polyoxypropylène-éthylènediamine, les polyéthylèneglycols, les polyoxyéthylène-esters, les polyesters modifiés, les dérivés de polyoxyéthylène-alcool et/ou les copolymères à blocs d'acides gras polyhydroxy-polyéthylèneglycol ou leurs combinaisons, la proportion de substance(s) tensioactive(s) étant de 0,1 à 15% en poids, par rapport au dispersant.

16. Procédé selon l'une quelconque des revendications 8 à 15, **caractérisé en ce qu'**on ajoute pendant le processus de dispersion 10 à 30% en volume, par rapport à la phase d'hydrogel, d'une base à 1 jusqu'à 20%, la base étant de préférence de l'ammoniaque.

17. Procédé selon l'une quelconque des revendications 8 à 16, **caractérisé en ce que** la proportion d'un ou de plusieurs composé(s) dans l'hydrosol, qui est/sont choisi(s) dans le groupe comprenant les composés métalliques, semi-métalliques ou leurs combinaisons, est de 1 à 20% en mole, les métaux du/des composé(s) étant de préférence choisis dans le groupe comprenant le zirconium, le titane, le cuivre, le cobalt, l'aluminium, le calcium, le potassium, le manganèse, le baryum, le magnésium et le zinc, et les semi-métaux du/des composé(s) étant de préférence choisis dans le groupe comprenant le bore et l'arsenic et les composés métalliques se trouvant de préférence sous forme de composés choisis dans le groupe comprenant les acétates, les fumarates, les alcoolates, l'acide éthylènediaminetétraacétique, l'acide nitrilotriacétique, les dérivés d'alkyle, les dérivés de porphine et les dérivés de carboxylate ou leurs combinaisons.

18. Procédé selon les revendications 8 à 17, **caractérisé en ce que** les particules d'hydrosol sont soumises en un laps de temps d'une à plusieurs heures à un traitement thermique à des températures > 250°C et sont ainsi transformées en particules de silicagel.

19. Procédé selon l'une quelconque des revendications 8 à 18, **caractérisé en ce que** les particules de silicagel sont transformées dans une autre étape de procédé avec des alkylalcoxysilanes substitués de formule générale X-(CH₂)ₙ-Si-(OR)₃, où X est choisi dans le groupe comprenant les radicaux aldéhyde, époxy, halogène, cyano, NH₂ ou mercapto, n = 1-6, de préférence 3, et R signifie un radical alkyle, trialkylsilyle ou H.

20. Utilisation des particules magnétiques selon l'une quelconque des revendications 1 à 7 pour la purification ou l'isolement de composés choisis dans le groupe comprenant des acides nucléiques, des protéines, des anticorps ou des fragments d'anticorps ainsi que des acides nucléiques biotinylés, des protéines biotinylées, des anticorps biotinylés ou des fragments d'anticorps biotinylés, comme échangeurs d'ions ou supports de chélate métallique, comme supports pour des biocatalyseurs tels que des protéines, des peptides, des enzymes, comme matrice d'encapsulation de protéines et de cellules ou comme adsorbants pour l'hémoperfusion.
